# EUROPEAN PATENT APPLICATION

(11) **EP 3 338 761 A1**
(43) Date of publication of application: **27.06.2018**
(21) Application number: 16205437.3
(22) Date of filing: 20.12.2016
(51) Int. Cl.: A61K 8/49, A61K 8/67, A61Q 19/00, A61K 8/06, A61K 8/39, A61K 8/86

(54) **COSMETIC COMPOSITIONS**

(71) Applicant: DSM IP Assets B.V., 6411 TE Heerlen (NL)
(72) Inventor: The designation of the inventor has not yet been filed
(74) Representative: Berg, Katja

(57) **Abstract**

The present invention relates to the use of ascorbyl phosphate and niacinamide to suppress the crystallization of allantoin in cosmetic compositions. Furthermore, the invention relates to cosmetic compositions comprising an ascorbyl phosphate, niacinamide, allantoin and a cosmetically acceptable carrier.

## Description

The present invention relates to the use of ascorbyl phosphate and niacinamide to suppress the crystallization of allantoin in cosmetic compositions. Furthermore, the invention relates to cosmetic compositions comprising an ascorbyl phosphate, niacinamide, allantoin and a cosmetically acceptable carrier.

Allantoin is recognized as an effective skin protectant active ingredient. Allantoin, however, is not very soluble in polar solvents such as water, glycerin propyleneglycol and practically insoluble in apolar solvents such as mineral oil. For formulators, its water solubility at concentrations of greater than 0.4 wt.-% at room temperature (about 20°C) is problematic, as above that concentration the aqueous solution has to be heated to at least 50°C to solubilize allantoin, which then recrystallizes upon cooling. As the recrystallization leads to a loss of activity as well as to aesthetically unpleasant appearance of the respective products, allantoin is generally used in amount of less than 0.5 wt.-% or, when used in higher concentrations, in dispersed form, which is, however, less active.

Thus, there is an ongoing need for agents, which suppress the recrystallization and thus allow the incorporation of higher amounts of solubilized allantoin into cosmetic compositions.

Surprisingly, it has been found that a combination of an ascorbyl phosphate and niacinamide significantly reduces the recrystallization of solubilized allantoin in water, which, in consequence, allows the incorporation of more solubilized, and thus active allantoin into cosmetic compositions.

Thus, in one aspect, the invention relates to cosmetic compositions comprising an ascorbyl phosphate, niacinamide and allantoin in the presence of a cosmetically acceptable carrier.

In another aspect, the present invention relates to a cosmetic compositions comprising
(a) 0.1 to 5 wt. %, preferably 0.3 to 4 wt. %, most preferably 0.5 to 3 wt.-% of an ascorbyl phosphate,
(b) 0.5 to 15 wt.-%, preferably 1 to 10 wt.-%, most preferably 2 to 5 wt.-% of niacinamide,
(c) 0.5 to 1 wt.-%, preferably 0.6 to 1 wt.-%, most preferably 0.7 to 1 wt.-% of allantoin, and
(d) a cosmetically acceptable carrier.

If nothing else is stated in this specification any given parts and percentages are per weight and based on the total weight of the composition.

The term allantoin as used herein refers to a compound of formula (1)

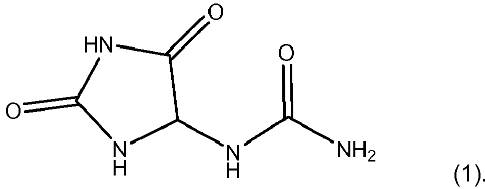

It is also known as 2,5-dioxo-4-imidazolidinyl urea, glyoxyldiureide respectively 5-ureido-hydantoine [CAS 97-59-6].

The term "ascorbyl phosphate" as used herein denotes metal salts of mono- and poly-phosphoric acid esters of ascorbic acid wherein the phosphorylated hydroxy group of the ascorbic acid molecule features one or more phosphoric acid (phosphate) units, and metal cations, e.g. sodium and/or magnesium or calcium ions, are also present. The term "poly" generally denotes 2 - 10, preferably 2 - 4, phosphate units. The ascorbyl phosphates may also be referred to in general as "ascorbyl (poly)phosphates" to embrace both mono- and polyphosphates. Typical ascorbyl phosphates for use in the present invention are L-ascorbic acid phosphate ester salts such as sodium ascorbyl phosphate, potassium ascorbyl phosphate, magnesium ascorbyl phosphate, calcium ascorbyl phosphate and sodium magnesium L-ascorbyl-2-monophosphate. The ascorbyl phosphates are essentially present in the form of a hydrate or a dihydrate. Commercially available ascorbyl phosphates comprise trisodium L-ascorbyl-2-monophosphate which is available as STAY-C^{®}50 from DSM Nutritional Products AG, (4303 Kaiseraugst, Switzerland) and magnesium L-ascorbyl phosphate (available from Showa Denko) and sodium magnesium L-ascorbyl-2-monophosphate. Preferred ascorbyl phosphates for all the purposes of the present invention are sodium or sodium magnesium or sodium calcium ascorbyl phosphate as well as mixtures thereof. Most preferred in all embodiments of the present invention is the use of trisodium L-ascorbyl-2-monophosphate dihydrate.

Niacinamide [CAS-Nr. 98-92-0] is one of the water-soluble B-complex vitamins which is e.g. available as Niacinamide PC or Niacinamide from DSM Nutritional Products AG, (4303 Kaiseraugst, Switzerland). Niacinamide is also referred to as nicotinamide or pyridine-3-carboxamide and is the amide of niacin (vitamin B₃).

The term 'cosmetic composition' as used herein refers to compositions, which are used to treat, care for or improve the appearance of the skin and/or the scalp. Particular advantageous cosmetic compositions according to the present invention are skin care preparations.

The term 'cosmetically acceptable carrier' (also referred to herein as carrier) refers to all vehicles/ carriers conventionally used in cosmetic compositions, i.e. which are suitable for topical application to the keratinous tissue, have good aesthetic properties, are compatible with the actives present in the composition, and will not cause any unreasonable safety or toxicity concerns. Such carriers are well-known to one of ordinary skill in the art.

The exact amount of carrier will depend upon the actual level of the actives (a) to (c) and any other optional ingredients that one of ordinary skill in the art would classify as distinct from the carrier (e.g., other active ingredients).

In an advantageous embodiment, the cosmetic compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water.

In a further embodiment, the invention relates to the use of an ascorbyl phosphate and niacinamide to solubilize allantoin water.

In yet another embodiment, the present invention relates to the use of an ascorbyl phosphate in combination with niacinamide for suppressing the (re-)crystallization of allantoin in cosmetic compositions.

The compositions according to the present invention can be prepared by conventional methods in the art. In a preferred embodiment, however, allantoin is solubilized in a mixture of water, niacinamide and an ascorbyl phosphate, before mixing with the residual ingredients.

Thus, the invention furthermore also relates a process (I) for the preparation of a cosmetic composition, said process comprising the step of
(a) solubilizing 0.5 to 1 wt.-%, preferably 0.6 to 1 wt.-%, most preferably 0.7 to 1 wt.-% of allantoin in an aqueous solution of 0.1 to 5 wt. %, preferably 0.3 to 4 wt. %, most preferably 0.5 to 3 wt.-% of an ascorbyl phosphate and 0.5 to 15 wt.-%, preferably 1 to 10 wt.-%, most preferably 2 to 5 wt.-% of niacinamide to form a solubilized allantoin solution, followed by
(b) admixing said solubilized allantoin solution with the cosmetic carrier and optionally further ingredients.

Preferably, the temperature in step (a) of process (I) is selected in the range of 30-85°C, such as preferably in the range of 40-60°c most preferably around 40°C.

In an alternative embodiment, the invention also relates a process (II) for the preparation of a cosmetic composition, said process comprising the step of
(a) solubilizing 0.5 to 1 wt.-%, preferably 0.6 to 1 wt.-%, most preferably 0.7 to 1 wt.-% of allantoin in an aqueous solution of 0.5 to 15 wt.-%, preferably 1 to 10 wt.-%, most preferably 2 to 5 wt.-% of niacinamide to form a solubilized allantoin solution at a elevated temperature, followed by
(b) admixing said solubilized allantoin solution with an aqueous solution of 0.1 to 5 wt. %, preferably 0.3 to 4 wt. %, most preferably 0.5 to 3 wt.-% of an ascorbyl phosphate after cooling to 30-50°C, preferably to 40°C, followed by
(c) admixing the thus obtained solution with the cosmetic carrier and optionally further ingredients.

Preferably, the elevated temperature in step (a) of process (II) is at least 50°C. More preferably, the temperature is selected in the range of 60-85°C, such as most preferably in the range of 70-80°C.

In a particular preferred embodiment, the pH of the aqueous solution prepared in step (a) is at least 8.0, more preferably, the pH is selected in the range of 8.0 to 8.5, most preferably in the range of 8.0 to 8.2. If necessary, the pH can be adjusted by well-known methods in the art using a cosmetically acceptable base such as aqueous hydroxide or a cosmetically acceptable acid such as e.g. citric acid.

In all embodiments of the present invention, preferably the amount of the ascorbyl phosphate as well as the amount of niacinamide is higher than the amount of allantoin. Advantageously, the amount of ascorbyl phosphate as well as the amount of niacinamide is independently of each other at least 1.5 times the amount of allantoin used, more preferably at least 3 times. In all embodiments of the present invention, it is furthermore preferred, that the amount of niacinamide is equal or higher than the amount of the ascorbyl phosphate. In particular, the ratio of niacinamide to the ascorbyl phosphate is selected in the range of about 1:1 to 25:1, more advantageously in the range of about 1:1 to 15:1, most advantageously in the range of about 1:1 to 10:1, such as in the range of about 1:1 to 5:1.

It is further preferred that the total amount of the ascorbyl phosphate and niacinamide used in the embodiments of the present invention is at least 3 wt.-%. Preferably the total amount of the ascorbyl phosphate and niacinamide is selected in the range of 5 to 30 wt.-%, more preferably in the range of 5 to 20-wt. %, most preferably in the range of 5 to 15 wt.-%.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions according to the present invention can be in a wide variety of forms. Non limiting examples include simple solutions (e.g. aqueous, organic solvent, or oil based), emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. of oil-in-water-in oil (O/W/O) or water-in-oil-in-water (W/O/W) type) or pickering emulsions), as well as solid forms (e.g. hydrogels, alcoholic gels, lipogels, sticks, flowable solids, or amorphous materials).

These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If the composition is an emulsion, such as in particular an O/W-, W/O-, Si/W-, W/Si-, O/W/O-, W/O/W- or a pickering emulsion, then the amount of the oily phase present in such cosmetic emulsions is preferably at least 10 wt.%, such as in the range of 10 to 60 wt.-%, preferably in the range of 15 to 50 wt.-%, most preferably in the range of 15 to 40 wt.-%, based on the total weight of the composition.

In one embodiment, the compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol® A from DSM Nutritional Products Ltd.), diethanolamine cetyl phosphate (e.g. as Amphisol® DEA from DSM Nutritional Products Ltd.), potassium cetyl phosphate (e.g. as Amphisol® K from DSM Nutritional Products Ltd.), sodium cetearylsulfate, sodium glyceryl oleate phosphate, hydrogenated vegetable glycerides phosphate and mixtures thereof. Further suitable emulsifiers are polyalkylene glycol ethers, sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the composition.

Particular suitable O/W emulsifiers to be used in the compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate as well as polyalkylene glycol ethers such as in particular polyethylene stearyl ethers such as Steareth-2 and Steareth 21.

A particular suitable class of O/W emulsifier to be used in the compositions according to the invention are polyalkyleneglycolethers. Particularly preferred O/W emulsifier in all emodiments of the present invention are the stearyl ethers of polyethyleneglycol, such as most preferably Steareth-2 (Polyoxyethylen (2) stearylether) or Steareth-21 (Polyoxyethylen (21) stearylether) as well as mixtures thereof. Such polyalkyleneglycolethers emulsifiers are e.g. commercially available under the Brij tradename at Croda.

In one particular embodiment, the invention relates to compositions with all the definitions and preferences given herein in the form of O/W emulsions comprising an oily phase dispersed in an aqueous phase in the presence of at least one O/W emulsifier wherein the at least one O/W emulsifier is a polyalkylene glycol ether such as preferably Steareth-2 and/ or Steareth-21. The amount of water used in such O/W emulsions is preferably at least 30 wt.-%, more preferably the amount of water is selected in the range of 30 to 80 wt.-%, most preferably in the range of 40 to 70 wt.-%, such as in the range of 50 to 60 wt.-%.

In another particular advantageous embodiment, the cosmetic compositions according to the present invention are clear serums. Such clear serums preferably consist essentially of allantoin, niacinamide, an ascorbylphosphate, water, a thickener and a preservative. It is furthermore particularly preferred that the thickener is xanthan gum.

The cosmetic compositions according to the present invention advantageously comprise a preservative. Particular suitable preservatives in all embodiments of the present invention are phenoxyethanol and ethylhexylglycerin as well as mixtures thereof. When present, the preservative is preferably used in an amount of 0.1 to 2 wt.-%, more preferably in an amount of 0.5 to 1.5 wt.-%, based on the total weight of the composition.

The compositions according to the invention in general have a pH in the range of 3 to 10, preferably a pH in the range of 4 to 8 and most preferably a pH in the range of 5 to 8. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The following examples are provided to further illustrate the compositions and effects of the present invention. These examples are illustrative only and are not intended to limit the scope of the invention in any way.

### Example 1: Solubility of Allantoin

The solubility of Allantoin was assessed in aqueous niacinamide (Niacinamide PC, DSM Nutritional Products) / sodium ascorbyl phosphate (STAY-C^{®} 50, DSM Nutritional Products) solutions in different concentrations. Then the solutions were stored at RT for 3 days, and then assessed visually at day 1 and 3 for crystal formation.

After the addition of the respective amounts of Niacinamide PC and STAY-C^{®} 50 as indicated in table 1 to water (total amount 100 g), the respective mixtures were stirred with a magnetic stirrer at 40°C until STAY-C^{®} 50 and Niacinamide PC were completely dissolved. Then, the respective amount of allantoin was added (total amount of each sample was 100 g) until completely dissolved as well. Then the samples were cooled to RT and stored. The samples were visually assessed after 1 and 3 days for crystal formation. The results are presented in table 1.

**Table 1**

| **#** | **Allantoin** | **Niacinamide PC** | **Stay-C 50** | **visual assessment after** | | **ratio** | **pH** |
|---|---|---|---|---|---|---|---|
| | **[wt.-%]** | | | **1 day** | **3 days** | | |
| 1 | 1 | 0 | 0 | crystals | crystals | 0.0 | n.a. |
| 2 | 1 | 0 | 0 | ok | crystals - | 0.0 | 8.26 |
| 3 | 1 | 0 | 1 | crystals | crystals | 0.0 | n.a. |
| 4 | 1 | 0 | 3 | ok | crystals | 0.0 | 8.01 |
| 5 | 1 | 1 | 0 | crystals | crystals | 0.0 | n.a. |
| 6 | 1 | 4 | 0 | crystals | crystals | 0.0 | n.a. |
| 7 | 1 | 10 | 0 | ok | crystals | 0.0 | 6.2 |
| 8 | 0.5 | 3 | 3 | ok | ok | 1.0 | n.a. |
| 9 | 0.7 | 3 | 3 | ok | ok | 1.0 | 8.1 |
| 10 | 0.8 | 3 | 3 | ok | ok | 1.0 | 8.03 |
| 11 | 0.9 | 3 | 3 | ok | ok | 1.0 | 8.04 |
| 12 | 1 | 3 | 3 | ok | ok | 1.0 | 8.1 |
| 13 | 1 | 5 | 3 | ok | ok | 1.7 | 8.03 |
| 14 | 1 | 10 | 3 | ok | ok | 3.3 | 8.1 |

As can be seen from the results presented in table 1 the solubility of allantoin in water can be significantly enhanced by STAY-C^{®} 50 and Niacinamide PC.

### Example 2: Preparation of a clear serum

Procedure: Mix Phase A at max. 40°C
When an almost clear solution is obtained, let cool down to RT. Adjust the pH if necessary to a pH of about 7.95 with B.

| **Phase** | INCI Name | **1 (Invention)** | **2 (Reference)** |
|---|---|---|---|
| A | Aqua | 89.7 | 97.7 |
| | Xanthan gum | 0.3 | 0.3 |
| | Phenoxyethanol, Ethylhexylglycerin | 1 | 1 |
| | Niacinamide | 5 | - |
| | Sodium ascorbyl phosphate | 3 | - |
| | Allantoin | 1 | 1 |
| B | Sodium hydroxide | - | 0.63 |
| pH | | 7.95 | 7.96 |
| **Visual appearance** | | | |
| immediately after preparation | | No crystals | No crystals |
| after 2h | | No crystals | Crystals |
| after 8h | | No crystals | Crystals |
| after 24h | | No crystals | Crystals |

### Example 3: Emulsion

Procedure: Mix phase A and melt it by heating under stirring up to 75°C
Phase B: Predispers Sclerotium Gum and Xanthan Gum in Glycerine, add preservative and then under stirring slowly the water and the other ingredients and heat up to 75°C
Pour A to B under stirring, homogenize 1.5 min at 13'000 rpm, let cool down to RT under stirring
Mix C under stirring at RT and add it to AB under stirring below 40°C, homogenize again 1 min at 11'000 rpm
Adjust the pH if necessary to pH of about 8.

| Emulsion | | 0.5% Allantoin | | 0.75% Allantoin | | 1.0% Allantoin | |
|---|---|---|---|---|---|---|---|
| | | **3** | **4** | **5** | **6** | **7** | **8** |
| **Phase** | INCI Name | **Inv.** | **Ref.** | **Inv.** | **Ref.** | **Inv.** | **Ref.** |
| A | Steareth-21 | 2 | 2 | 2 | 2 | 2 | 2 |
| | Steareth-2 | 2 | 2 | 2 | 2 | 2 | 2 |
| | Cetearyl alcohol | 2 | 2 | 2 | 2 | 2 | 2 |
| | Glyceryl myristate | 2 | 2 | 2 | 2 | 2 | 2 |
| | Caprylic/capric triglyceride | 10 | 10 | 10 | 10 | 10 | 10 |
| | C12-15 Alkyl benzoate | 10 | 10 | 10 | 10 | 10 | 10 |
| B | Phenoxyethanol, Ethylhexylglycerin | 1 | 1 | 1 | 1 | 1 | 1 |
| | Aqua | Ad 100 | | | | | |
| | Disodium EDTA | 0.2 | 0.2 | 0.2 | 0.2 | 0.2 | 0.2 |
| | Glycerin | 4 | 4 | 4 | 4 | 4 | 4 |
| | Xanthan gum | 0.3 | 0.3 | 0.3 | 0.3 | 0.3 | 0.3 |
| | Scleroticum gum | 0.2 | 0.2 | 0.2 | 0.2 | 0.2 | 0.2 |
| | Allantoin | 0.5 | 0.5 | 0.75 | 0.75 | 1 | 1 |
| | Niacinamide | 5 | - | 5 | - | 5 | - |
| C | Sodium ascorbyl phosphate | 3 | - | 3 | - | 3 | - |
| | Aqua | 10 | - | 10 | - | 10 | - |
| D | Sodium hydroxide | - | 0.5 | - | 0.59 | - | 0.68 |
| | pH | 7.94 | 7.97 | 7.89 | 8.01 | 7.85 | 7.87 |
| **Microscopy assessment for crystal formation** | | | | | | | |
| Directly after preparation | | none | none | none | none | none | none |
| After 20h | | none | none | none | crystals | none | crystals |
| After 5 days | | none | crystals | none | crystals | none | crystals |

## Claims

1. A cosmetic composition comprising
(a) 0.1 to 5 wt. %, preferably 0.3 to 4 wt. %, most preferably 0.5 to 3 wt.-% of an ascorbyl phosphate,
(b) 0.5 to 15 wt.-%, preferably 1 to 10 wt.-%, most preferably 2 to 5 wt.-% of niacinamide,
(c) 0.5 to 1 wt.-%, preferably 0.6 to 1 wt.-%, most preferably 0.7 to 1 wt.-% of allantoin, and
(d) a cosmetically acceptable carrier.

2. The cosmetic according to claim 1, wherein the ascorbyl phosphate is selected from the group consisting of trisodium L-ascorbyl-2-monophosphate, magnesium L-ascorbyl phosphate and sodium magnesium L-ascorbyl-2-monophosphate as well as mixtures thereof.

3. The cosmetic according to claim 1, wherein the ascorbyl phosphate is trisodium L-ascorbyl-2-monophosphate.

4. The cosmetic composition according to anyone of the preceding claims, wherein the carrier consists of at least 40 wt.-%, more preferably of at least 50 wt.-%, most preferably at least 55 wt.-% of water, such as in particular of about 55 to about 90 wt.-% of water, based on the total weight of the composition.

5. The composition according to anyone of the preceding claims, wherein the amount of the ascorbyl phosphate and the amount of niacinamide is at least 1.5 times, preferably at least three times the amount of allantoin.

6. The composition according to anyone of the preceding claims, wherein the ratio of niacinamide to the ascorbyl phosphate is selected in the range of about 1:1 to 25:1, more advantageously in the range of about 1:1 to 15:1, most advantageously in the range of about 1:1 to 10:1, such as in the range of about 1:1 to 5:1.

7. The composition according to anyone of the preceding claims, wherein the total amount of the ascorbyl phosphate and the niacinamide is at least 3 wt.-%, based on the total weight of the composition.

8. The composition according to anyone of the preceding claims, wherein the composition is in the form of O/W emulsions comprising an oily phase dispersed in an aqueous phase in the presence of at least one polyalkylene glycol ether emulsifier, preferably in the presence of Steareth-2 and/ or Steareth-21.

9. The composition according to anyone of the proceeding claims, wherein the composition is a clear serum.

10. Use of an ascorbyl phosphate and niacinamide to solubilize allantoin in water.

11. Use of an ascorbyl phosphate and niacinamide for suppressing the crystallization of allantoin in cosmetic compositions.

12. The use according to claim 10 or 11, wherein the amount of the ascorbyl phosphate and the amount of niacinamide is at least 1.5 times the amount of allantoin.

13. A process for the preparation of a cosmetic composition, said process comprising the step of
(a) solubilizing 0.5 to 1 wt.-%, preferably 0.6 to 1 wt.-%, most preferably 0.7 to 1 wt.-% of allantoin in an aqueous solution of 0.1 to 5 wt. %, preferably 0.3 to 4 wt. %, most preferably 0.5 to 3 wt.-% of an ascorbyl phosphate and 0.5 to 15 wt.-%, preferably 1 to 10 wt.-%, most preferably 2 to 5 wt.-% of niacinamide to form an solubilized allantoin solution, followed by
(b) admixing said solubilized allantoin solution with a cosmetic carrier and optionally further ingredients.

14. A process according to claim 13, wherein the temperature in step (a) is selected in the range of 30-85°C, such as preferably in the range of 40-60°C most preferably around 40°C.

15. The process according to claim 13 or 14, wherein the pH of the aqueous solution prepared in step (a) is at least 8.0, more preferably selected in the range of 8.0 to 8.5, most preferably selected in the range of 8.0 to 8.2.
